# EUROPEAN PATENT APPLICATION

(11) **EP 4 678 175 A1**
(43) Date of publication of application: **14.01.2026**
(21) Application number: 24769822.8
(22) Date of filing: 06.03.2024
(51) Int. Cl.: A61K 31/519, A61P 35/00

(54) **USE OF PHARMACEUTICAL COMPOUND IN TREATMENT OF SOLID TUMORS**

(30) Priority: 10.03.2023 CN 202310231050
(71) Applicant: Shouyao Holdings (Beijing) Co., Ltd., Beijing 100176 (CN)
(72) Inventor: GAO, Yuhao, Beijing 100195 (CN); ZHANG, Kai, Beijing 100195 (CN); LU, Chang, Beijing 100195 (CN)
(74) Representative: f & e patent
(86) International application number: PCT/CN2024/080338
(87) International publication number: WO 2024/188129

(57) **Abstract**

This invention is in the field of pharmaceutical technology, and discloses the use of a pharmaceutical compound in the treatment of solid tumors, the compound is *N²*-(4-(4-(dimethyl amino)piperidin-1-yl)-2-methoxyphenyl)-*N⁴*-(2-(isopropylsulfonyl)phenyl)-7H-pyrrolo[2,3-d] pyrimidine-2,4-diamine, which can be used to treat CLIP1-LTK fusion-positive tumors, including non-small cell lung cancer.

## Description

### Cross-Reference

This application claims priority to Chinese Patent Application No. 202310231050. X, filed on March 10th, 2023, entitled "USE OF PHARMACEUTICAL COMPOUND IN THE TREATMENT OF SOLID TUMORS", the entire disclosure of which is incorporated herein by reference in its entirety.

### Technical Field

The present invention is in the field of pharmaceutical technology and relates to the use of a pharmaceutical compound in the treatment of solid tumors.

### Background Art

Lung cancer is one of the most aggressive malignant tumors. Among all types of cancer, lung cancer has the highest mortality rate, accounting for 19% of cancer-related deaths and 3% of global total deaths. Non-small cell lung cancer (NSCLC), accounting for about 85% of the total number of lung cancers, is the most common type of lung cancer. With the development of gene detection technology, more and more lung cancer driver genes have been found, and the number of targeted therapeutic drugs is increasing, which has entered the era of precise treatment.

However, in addition to the well-known targets such as EGFR and ALK, there is a further breakthrough in rare or rare targets such as KRAS, RET, MET, and ROS1. New drugs for rare targets have been approved for marketing successively, and the treatment of rare targets for lung cancer is gradually gaining acceptance. However, tumor driver genes are still not found in 10-20% of lung adenocarcinoma patients.

Hiroli Izumi et al. (Nature, 11, 2021) found that NSCLC was a new therapeutic target, CLIP1-LTK, and the probability of fusion mutation at NSCLC was 0.4%. Fusion-positive tumors were negative for other known oncogene driver genes, indicating that the CLIP1-LTK fusion is mutually exclusive of other established oncogenes. The above studies indicate that the CLIP1-LTK fusion is likely to be the oncogenic driver gene in NSCLC, but there are few reports on the small molecule inhibitors of this target, and it still needs more research results to confirm that the fusion is really the next target for precise treatment of lung cancer.

CN108276410A discloses a series of compounds targeting ALK, and in vitro enzymatic activity tests and cell proliferation inhibition tests show that said compounds can significantly inhibit the kinase activity of ALK, including compounds such as *N²*-(4-(4-(dimethylamino) piperidin-1-yl)-2-methoxyphenyl)-*N⁴*-(2-(isopropylsulfonyl)phenyl)-7H-pyrrolo[2,3-d] pyrimidine -2,4-diamine.

### Summary of the Invention

The inventors have discovered in a serendipitous experiment that *N²*-(4-(4-(dimethylamino) piperidin -1-yl)-2-methoxyphenyl)-*N⁴*-(2-(isopropylsulfonyl) phenyl)-7H-pyrrolo 2,3-d pyrimidine -2,4-diamine has a significant inhibitory effect on LTK tyrosine kinase and on tumor growth in animals that are positive for the CLIP1-LTK fusion protein.

The structure of *N²*-(4-(4-(dimethylamino) piperidin-1-yl)-2-methoxyphenyl)-*N⁴*-(2-(isopropylsulfonyl) phenyl)-7H-pyrrolo [2,3-d] pyrimidine -2,4-diamine is shown in Formula (I) below:

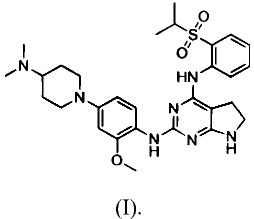

The present invention relates to the following.

It is an object of the present invention to provide a pharmaceutical composition including a compound of formula (I) or a pharmaceutically acceptable salt thereof, optionally together with a pharmaceutically acceptable carrier.

It is an object of the present invention to provide the use of a compound of formula (I), or a pharmaceutically acceptable salt thereof, or a composition as described above, in the manufacture of a drug for treating an LTK-mediated disease.

It is an object of the present invention to provide a method for treating an LTK-mediated disease, which includes administering to a subject a therapeutically effective amount of a compound of formula (I) or a pharmaceutically acceptable salt thereof, or a pharmaceutical composition as described above.

It is an object of the present invention to provide the use of a compound of formula (I) or a pharmaceutically acceptable salt thereof, or a pharmaceutical composition as described above, in the manufacture of a drug for treating a CLIP1-LTK fusion-positive disease.

It is an object of the present invention to provide a method for treating a CLIP1-LTK fusion-positive disease, which includes administering to a subject a therapeutically effective amount of a compound of formula (I) or a pharmaceutically acceptable salt thereof, or a pharmaceutical composition as described above.

In a preferred embodiment, the LTK-mediated disease or CLIP1-LTK fusion-positive disease is a solid tumor.

In a more preferred embodiment, the solid tumor is non-small cell lung cancer, anaplastic large cell lymphoma, inflammatory myofibroblastic tumor, nasopharyngeal carcinoma, breast cancer, colorectal cancer, diffuse large B-cell lymphoma, systemic histiocytosis, and neuroblastoma.

In a most preferred embodiment, the non-small cell lung cancer is LTK-mediated or CLIP1-LTK fusion-positive non-small cell lung cancer.

In a certain embodiment, a compound of formula (I) or a pharmaceutically acceptable salt thereof, or a pharmaceutical composition as described above, may also be administered in combination with a second agent that is a chemotherapeutic drug, a chemically targeted drug, or an antibody drug.

As used herein, the term "LTK" refers to leukocyte receptor tyrosine kinase (English name: Leukocyte Receptor Tyrosine Kinase). The term "CLIP1" refers to CAP-Gly domain containing linker protein 1 (English name: CAP-Gly Domain Containing Linker Protein 1).

As used herein, NIH3T3 cells refer to mouse embryonic fibroblast cell lines established by the United States National Institutes of Health (NIH). Baf3 cells refer to the mouse pro-B lymphocyte cell line.

### Brief Description of the Drawings

FIG. 1: inhibition effect of compounds of formula (I) on LTK kinase.
FIG. 2A: inhibitory activity of compounds of formula (I) on the proliferation of Baf3 cells expressing CLIP1-LTK fusions.
FIG. 2B: inhibitory activity of compounds of formula (I) on the proliferation of NIH3T3 cells expressing CLIP1-LTK fusions.
FIG. 3A: effect of compounds of formula (I) on tumor growth in CLIP1-LTK/Baf3 tumor-bearing mice.
FIG. 3B: effect of compounds of formula (I) on body weight of CLIP1-LTK/Baf3 tumor-bearing mice.
FIG. 4A: effect of compounds of formula (I) on tumor growth in CLIP1-LTK/NIH3T3 tumor-bearing mice.
FIG. 4B: effect of compounds of formula (I) on body weight of CLIP1-LTK/NIH3T3 tumor-bearing mice.

### Detailed Description of the Invention

The present invention is further illustrated, but not limited, by the following examples and the accompanying drawings.

### Example 1

LTK kinase inhibitory activity assay of the compound of Formula (I) (*N²*-(4-(4-(dimethylamino) piperidin-1-yl)-2-methoxyphenyl)-*N⁴*-(2-(isopropylsulfonyl) phenyl)-7H-pyrrolo [2,3-d] pyrimidine -2,4-diamine).

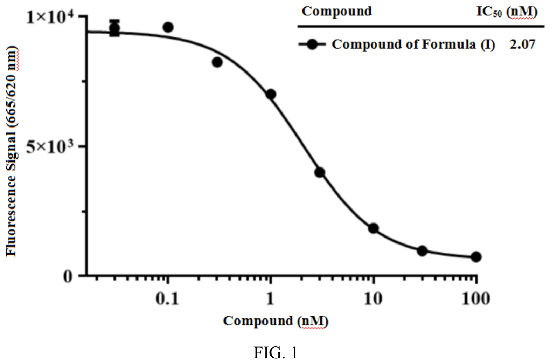

A homogeneous time-resolved fluorescence (HTRF) assay system was used to establish a LTK kinase activity detection platform for the compound activity assay. Preparation of reaction buffer: 50 mM HEPES, pH 7.5; 2 µM Na₃VO₄; 0.001% Tween-20; 5 mM MgCl₂; 1 mM DTT; 33.3 nM SEB (available from Cisbio) and 0.01% BSA. The compound was diluted in a 3-fold gradient starting at 10 µM with 10% DMSO in reaction buffer (for a total of 10 concentrations). 2 µL of the diluted compound at each concentration was added to 48 µL of reaction buffer and mixed well. 2.5 µL was added to a 384-well plate (OptiPlate-384, available from PerkinElmer), followed by 5 µL of GST-LTK (498-796 aa, final concentration being 0.1 nM), mixed well by centrifugation, and the reaction was initiated by the addition of 2.5 µL of ATP (final concentration being 10 µM) and TK peptide substrate cocktail (final concentration being 1 µM, available from Cisbio) in a total reaction volume of 10 µL. The 384-well plate was placed in an incubator at 23°C for 1 h and then stopped by the addition of 5 µL TK Antibody (available from Cisbio), 5 µL Streptavidin-XL665 (available from Cisbio). After a further 1 h incubation in the incubator, the fluorescence intensity was read on an Envision machine (available from PerkinElmer) with excitation at 320 nm and emission detected at 665 nm and 620 nm; the ratio was the activity signal value for LTK kinase. The signal value of the compound at each concentration was determined, and the IC₅₀ value of the compound for LTK enzymatic activity inhibition was calculated using GraphPad Prism software. As shown in FIG. 1, the compound had strong inhibitory activity against LTK kinase with an IC₅₀ value of 2.07 nM for inhibition of LTK kinase activity.

Unexpectedly, the applicant tested other specific compounds disclosed in the patent document CN108276410A by using the above-mentioned method, and the specific compounds in examples 2, 13-14, 16-17, 18 and 20 in the patent have poor inhibitory ability on LTK kinase activity, with IC₅₀ values being greater than 50 nM, failing to effectively inhibit the activity of LTK kinase.

### Example 2

Cell proliferation inhibitory activity assay of compounds of formula (I).

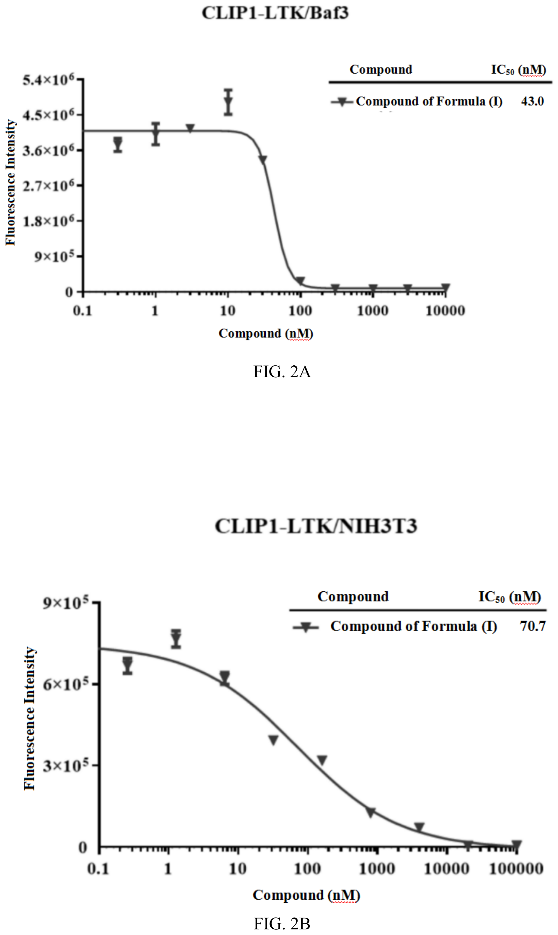

Lentivirus infection was used to construct LTK fusion cell lines CLIP1-LTK/Baf3 and CLIP1-LTK/NIH3T3. CLIP1-LTK/Baf3 cells were cultured in suspension using RPMI-1640 medium plus 10% fetal bovine serum (FBS, available from Biological Industries, BI) and 1% Penicillin/Streptomycin double antibody (P/S, available from Life Technology); CLIP1-LTK/NIH3T3 cells were adherent cultured using DMEM medium plus 10% fetal bovine serum (FBS, available from Biological Industries, BI) and 1% Penicillin/Streptomycin double antibody (P/S, available from Life Technology). The cells were cultured in a cell culture incubator at 37°Cand 5% CO₂. The day before compound detection, CLIP1-LTK/Baf3 cells were plated in 96-well plates (available from Coming) at a density of 2000 cells/195 µL/well; CLIP1-LTK/NIH3T3 cells were plated in 96-well plates at a density of 1000 cells/195 µL/well. Compounds were serially diluted 3-fold (11 concentrations total) starting at 10 mM with 100% DMSO after 24 h, and then 2 µL of each concentration was added to 48 µL of serum-free medium for further dilution. 5 µL of the diluted compound at each concentration was added to the cell culture plate and placed in a cell culture incubator for an additional 3 days. After 3 days, for CLIP1-LTK/Baf3 cells, 45 µL Cell-Titer Glo reagent (available from Promega) was added directly to the culture medium; for CLIP1-LTK/NIH3T3 cells, 25 µL Cell-Titer Glo reagent was added after media exhaustion. The cells were incubated at room temperature for 5-10 min. Fluorescence signal values were read on an Envision machine, and IC₅₀ values for the inhibition of cell proliferation by the compounds were calculated using GraphPad Prism software. As shown in FIG. 2A and FIG. 2B, the compounds of the present invention had significant inhibitory activity against the proliferation of Baf3 and NIH3T3 cells expressing the CLIP1-LTK fusion, with IC₅₀ values of 43.0 nM and 70.7 nM, respectively.

### Example 3

### In vivo pharmacodynamic evaluation of compounds

Subcutaneous xenograft models in CLIP1-LTK/Baf3 and CLIP1-LTK/NIH3T3 mice were established by subcutaneous injection of 2.0 x 10⁶ CLIP1-LTK/Baf3 cells or 7.5 x 10⁶ CLIP1-LTK/NIH3T3 cells and 30% Matrigel (BD) in 0.1 mL PBS suspension on both sides of the back of 6-week-old SPF grade BALB/c-nude female mice (available from Beijing Vital River Laboratory Animal Technology Co., Ltd.), respectively.

When the average tumor volume reached 100-200 mm³, tumor-bearing mice with regular shape and uniform size were selected to enter the experiment, and were randomly divided into groups according to tumor volume and body weight. The experimental animals were orally administered with vehicle and compounds at different concentrations, respectively, starting on the day of grouping, once a day for 9 consecutive days. During the experiment, the long diameter (cm) and short diameter (cm) of the tumor were measured by vernier caliper, and the volume of the tumor was calculated by formula. Tumor volume (cm³) = 0.5 x long diameter (cm) x short diameter (mm)². Tumor diameter was measured 3 times a week, tumor volume was calculated, and a tumor growth curve was drawn. At the end of the experiment, the mice were sacrificed, and tissues such as the tumor were collected, photographed, and weighed.

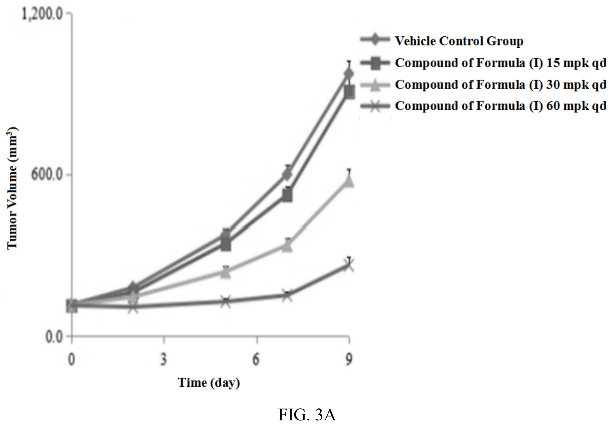

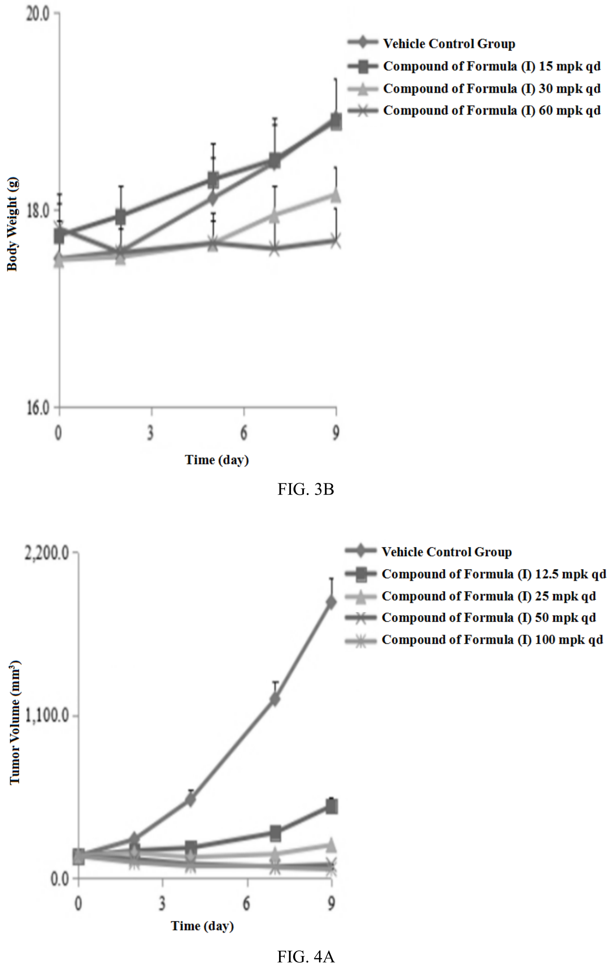

Among them, the results of the experiment against CLIP1-LTK/Baf3 tumor-bearing mice, in which the growth of the tumor was inhibited in a dose-dependent manner, are shown in FIGs. 3A and 3B. FIG. 3A was collated to obtain the effect of compounds on tumor volume growth inhibition rate (Table 1) and relative tumor proliferation rate (Table 2) in CLIP1-LTK/Baf3 tumor-bearing mice. In terms of compound safety, no drug-induced death or body weight change was observed in the tumor-bearing mice in the compound treatment group, and no significant abnormality was observed in the general state of each tumor-bearing mouse, indicating that the compound was well tolerated (FIG. 3B).

**Table 1**

| **Grouping** | **Tumor Growth Inhibition rate (%)** | | | |
|---|---|---|---|---|
| | Day 2 | Day 5 | Day 7 | Day 9 |
| 15 mg/kg, once a day | 22.4 | 11.1 | 15.1 | 7.3 |
| 30 mg/kg, once a day | 52.2 | 52.0 | 53.6 | 46.2 |
| 60 mg/kg, once a day | 108.5 | 94.5 | 92.1 | 82.5 |

| Table 2 | | | | |
|---|---|---|---|---|
| **Grouping** | **Relative Tumor Proliferation Rate (%)** | | | |
| | Day 2 | Day 5 | Day 7 | Day 9 |
| 15 mg/kg, once a day | 92.9 | 93.0 | 88.9 | 94.6 |
| 30 mg/kg, once a day | 82.0 | 62.8 | 57.0 | 59.1 |
| 60 mg/kg, once a day | 62.5 | 34.4 | 25.6 | 27.0 |

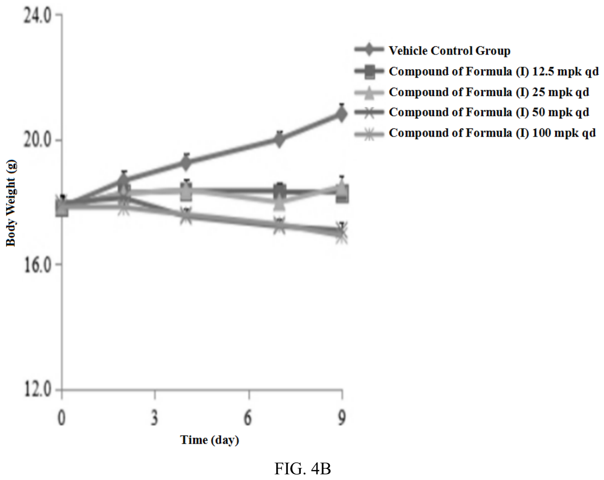

Among them, the results of the experiment against CLIP1-LTK/NIH3T3 tumor-bearing mice, in which the growth of the tumor was inhibited in a dose-dependent manner, are shown in FIGs. 4A and 4B. FIG. 4A was collated to obtain the effect of compounds on tumor volume growth inhibition rate (Table 3) and relative tumor proliferation rate (Table 4) in CLIP1-LTK/NIH3T3 tumor-bearing mice. In terms of compound safety, no drug-induced death or body weight change was observed in the tumor-bearing mice in the compound treatment group, and no significant abnormality was observed in the general state of each tumor-bearing mouse, indicating that the compound was well tolerated (FIG. 4B).

**Table 3**

| **Grouping** | **Tumor growth inhibition rate (%)** | | | |
|---|---|---|---|---|
| | Day 2 | Day 4 | Day 7 | Day 9 |
| 12.5 mg/kg, once a day | 68.6 | 85.9 | 85.3 | 80.4 |
| 25 mg/kg, once a day | 83.3 | 102.6 | 99.0 | 95.8 |
| 50 mg/kg, once a day | 117.3 | 113.7 | 106.7 | 103.4 |
| 100 mg/kg, once a day | 138.7 | 117.9 | 107.3 | 105.2 |

| Table 4 | | | | |
|---|---|---|---|---|
| **Grouping** | **Relative Tumor Proliferation Rate (%)** | | | |
| | Day 2 | Day 4 | Day 7 | Day 9 |
| 12.5 mg/kg, once a day | 70.0 | 38.4 | 24.7 | 25.9 |
| 25 mg/kg, once a day | 64.8 | 26.5 | 13.1 | 11.7 |
| 50 mg/kg, once a day | 50.5 | 19.0 | 6.4 | 4.8 |
| 100 mg/kg, once a day | 40.8 | 16.1 | 6.0 | 3.2 |

While particular embodiments of the present invention have been described above with reference to specific embodiments thereof, it is not intended to limit the scope of the present invention. It will be apparent to those skilled in the art that various modifications and variations can be made in the present invention without departing from the spirit or scope of the present invention.

## Claims

1. Use of *N²*-(4-(4-(dimethylamino) piperidin-1-yl)-2-methoxyphenyl)-*N⁴*-(2-(isopropyl sulfonyl)phenyl)-7H-pyrrolo [2,3-d] pyrimidine -2,4-diamine or a pharmaceutically acceptable salt thereof, or a therapeutic agent comprising any one of the foregoing, in the manufacture of a drug for treating an LTK-mediated disease.

2. A method for treating an LTK-mediated disease, **characterized by** comprising administering to a subject a therapeutically effective amount of *N²*-(4-(4-(dimethylamino) piperidin-1-yl)-2-methoxyphenyl)-*N⁴*-(2-(isopropylsulfonyl)phenyl)-7H-pyrrolo[2,3-d]pyrimidine-2,4-diamine or a therapeutic agent comprising *N²*-(4-(4-(dimethylamino)piperidin-1-yl)-2-methoxyphenyl)-*N⁴*-(2-(isopropylsulfonyl) phenyl)-7H-pyrrolo [2,3-d] pyrimidine -2,4-diamine.

3. Use of *N²*-(4-(4-(dimethylamino) piperidin-1-yl)-2-methoxyphenyl)-N4-(2-(isopropyl sulfonyl)phenyl)-7H-pyrrolo [2,3-d] pyrimidine -2,4-diamine or a pharmaceutically acceptable salt thereof, or a therapeutic agent comprising any one of the foregoing, in the manufacture of a drug for treating a CLIP1-LTK fusion-positive disease.

4. A method for treating a CLIP1-LTK fusion-positive disease, **characterized by** comprising administering to a subject a therapeutically effective amount of N2-(4-(4-(dimethylamino) piperidin-1-yl)-2-methoxyphenyl)-N4-(2-(isopropylsulfonyl)phenyl)-7H-pyrrolo[2,3-d]pyrimidine -2,4-diamine or a therapeutic agent comprising N2-(4-(4-(dimethylamino) piperidin-1-yl)-2-methoxyphenyl)-N4-(2-(isopropylsulfonyl) phenyl)-7H-pyrrolo [2,3-d] pyrimidine -2,4-diamine.

5. A therapeutic agent for an LTK-mediated disease or a CLIP1-LTK fusion-positive disease, **characterized by** comprising *N²*-(4-(4-(dimethylamino)piperidin-1-yl)-2-methoxyphenyl)-*N⁴*-(2-(isopropylsulfonyl) phenyl)-7H-pyrrolo [2,3-d] pyrimidine -2,4-diamine or a pharmaceutically acceptable salt thereof, and a pharmaceutically acceptable excipient.

6. The use, method, or therapeutic agent according to any one of claims 1 to 5, **characterized in that** the *N²*-(4-(4-(dimethylamino) piperidin -1-yl)-2-methoxyphenyl)-*N⁴*-(2-(isopropylsulfonyl) phenyl)-7H-pyrrolo [2,3-d] pyrimidine -2,4-diamine has a structure shown in Formula I below:

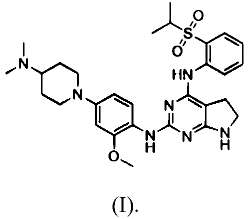

7. The use, method, or therapeutic agent according to any one of claims 1 to 5, **characterized in that** the LTK-mediated disease or CLIP1-LTK fusion-positive disease is a solid tumor.

8. The use, method, or therapeutic agent according to claim 7, **characterized in that** the solid tumors are non-small cell lung cancer, anaplastic large cell lymphoma, inflammatory myofibroblastic tumor, nasopharyngeal carcinoma, breast cancer, colorectal cancer, diffuse large B-cell lymphoma, systemic histiocytosis, and neuroblastoma.

9. The use, method, or therapeutic agent according to claim 8, **characterized in that** the non-small cell lung cancer is LTK-mediated or CLIP1-LTK fusion-positive non-small cell lung cancer.

10. The therapeutic agent according to any one of claims 5 to 9, **characterized in that** the therapeutic agent is administered in combination with a second agent which is a chemotherapeutic drug, a chemically targeted drug, or an antibody drug.
